# FASCICULE DE BREVET EUROPEEN

(11) **EP 1 024 123 B1**
(45) Date de publication et mention de la délivrance du brevet: **28.04.2004**
(21) Numéro de dépôt: 00400051.9
(22) Date de dépôt: 11.01.2000
(51) Int. Cl.: C07C 6/04

(54) **Procédé de métathèse des oléfines en présence d'un agent stabilisant du catalyseur**
Verfahren zur Metathesis von Olefinen in Gegenwart von einem Stabilisierungsmittel für den Katalysator
Process for the metathesis of olefins in presence of a stablising agent for the catalyst

(30) Priorité: 29.01.1999 FR 9901277
(43) Date de publication de la demande: 02.08.2000
(73) Titulaire: Institut Français du Pétrole, 92852 Rueil-Malmaison Cedex (FR)
(72) Inventeur: Commereuc, Dominique, 92190 Meudon (FR); Mikitenko, Paul, 78590 Noisy le Roi (FR)

(56) Documents cités:
- EP-A- 0 769 323

## Description

La présente invention concerne un procédé de métathèse d'oléfines en présence d'un catalyseur et d'un agent stabilisant injecté dans le milieu réactionnel pour réduire la désactivation du catalyseur, l'injection est réalisée de façon continue ou discontinue pendant tout le temps de la réaction de métathèse. Les oléfines susceptibles de réagir sont des monooléfines ayant de 2 à 30 atomes de carbone, des cyclooléfines ayant de 3 à 20 atomes de carbone, des polyoléfines ayant de 4 à 30 atomes de carbone et des cyclopolyoléfines ayant de 5 à 30 atomes de carbone ; l'agent stabilisant injecté dans le milieu réactionnel est un composé de l'aluminium X_{q}AlR'ᵣ, dans lequel X est un radical choisi dans le groupe formé par les alkoxydes et les aryloxydes RO-, les sulfures RS- et les amidures R₂N-, R est un reste hydrocarbyle contenant de 1 à 40 atomes de carbone, R' est un reste alkyle contenant de 1 à 20 atomes de carbone, q et r sont égaux à 1 ou 2 de telle façon que la somme q+r soit égale à 3 ; et le catalyseur contient du rhénium. L'invention s'applique très avantageusement lorsque la réaction est conduite dans une colonne de distillation comportant au moins une zone réactive catalytique, appelée dans la suite colonne de distillation réactive.

La métathèse des oléfines est une réaction équilibrée qui consiste en une redistribution statistique des groupements alkylidènes des oléfines qui sont mises en présence. Elle présente un grand intérêt pratique, par exemple pour le rééquilibrage entre elles des oléfines légères issues du craquage à la vapeur ou du craquage catalytique (FCC), ou éventuellement d'une réaction de Fischer-Tropsch, telles que l'éthylène, le propylène, les butènes ou les pentènes. De façon générale, elle est catalysée par les composés du tungstène, du molybdène ou du rhénium. A cause de sa nature statistique, la réaction fournit comme produit un mélange en général complexe, qu'il est nécessaire de fractionner pour pouvoir recycler au réacteur les réactifs non convertis afin d'augmenter leur taux de conversion.

La réaction de métathèse est habituellement réalisée soit en batch, soit en continu, en utilisant un réacteur dans lequel le catalyseur est sous la forme d'un lit fixe, d'un lit agité, d'un lit mobile, ou d'un lit fluidisé. A la fin de la réaction (en batch), ou à la sortie du réacteur (en continu), l'effluent est dirigé vers des colonnes de distillation pour séparer les produits et les réactifs non transformés. Les schémas des procédés de métathèse sont donc en général complexes en raison de la nature équilibrée de la réaction.

L'utilisation d'une colonne de distillation réactive dans laquelle la réaction de métathèse et la séparation des réactifs et des produits se fait simultanément, peut alors, dans son principe, présenter de nombreux avantages, ainsi qu'il est décrit dans le brevet US 4 709 115 pour la dismutation du butène-1. Dans ce cas, la séparation in situ, d'une part des réactifs et des produits, d'autre part des produits entre eux, permet d'augmenter de façon notable la conversion des réactifs, et aussi la sélectivité de la réaction en diminuant les possibilités de réactions secondaires des produits entre eux. La possibilité d'utilisation d'une distillation réactive en métathèse est également mentionnée dans le brevet EP 832 867.

Cependant, on a constaté que les catalyseurs de métathèse, qu'ils soient à base de tungstène, de molybdène ou de rhénium, se désactivent rapidement au cours du temps, et nécessitent donc de fréquentes régénérations. La méthode de régénération diffère peu selon le métal et comporte presque toujours au moins une phase de calcination du catalyseur à haute température, par exemple entre 400 et 1000 °C. Ceci ne pose pas de problème particulier pour la mise en oeuvre lorsque le catalyseur est placé en lit fixe dans un réacteur conçu en conséquence, ou bien transféré du réacteur dans un régénérateur grâce à un lit mobile ou un lit fluidisé. Par contre, la fréquence des régénérations réduit considérablement la productivité de l'installation.

La nécessaire mise en oeuvre de régénérations fréquentes est au contraire un problème pratiquement insoluble si le catalyseur est placé à l'intérieur d'une colonne de distillation comportant des plateaux ou du garnissage destinés à favoriser le contact liquide-vapeur. La technologie de la distillation réactive n'est ainsi pas applicable au niveau industriel avec les catalyseurs de métathèse conventionnels.

Il a maintenant été trouvé de façon inattendue que l'injection en continu, séparément ou avec la charge de métathèse, d'un composé de l'aluminium X_{q}AlR'ᵣ, permet de réduire considérablement la désactivation du catalyseur. Ainsi, il est possible d'envisager la mise en oeuvre de la métathèse, soit dans un réacteur conventionnel avec des régénérations beaucoup plus espacées, soit dans une colonne de distillation réactive.

L'invention concerne donc précisément un procédé de métathèse d'oléfines en présence d'un catalyseur et d'un agent stabilisant injecté dans le milieu réactionnel. Cela signifie que l'agent stabilisant est injecté pendant tout le temps de déroulement du procédé de métathèse, l'injection ayant lieu de façon continue ou discontinue.

Un objet de l'invention est plus précisément un procédé de métathèse des oléfines, dans lequel il est injecté dans le milieu réactionnel un composé de l'aluminium X_{q}AlR'ᵣ, dans lequel X est un radical choisi dans le groupe formé par les alkoxydes et les aryloxydes RO-, les sulfures RS- et les amidures R₂N-, R est un reste hydrocarbyle contenant de 1 à 40 atomes de carbone, R' est un reste alkyle contenant de 1 à 20 atomes de carbone, q et r sont égaux à 1 ou 2 de telle façon que la somme q+r soit égale à 3.

Le composé d'aluminium stabilisant répond à la formule générale X_{q}AlR'ᵣ. Dans cette formule, X est un radical choisi dans le groupe formé par les alkoxydes et les aryloxydes RO-, les sulfures RS- et les amidures R₂N-, R est un reste hydrocarbyle contenant de 1 à 40 atomes de carbone, par exemple alkyle, cycloalkyle, alkényle, aryle, aryle ou cycloalkyle substitués, de préférence un reste hydrocarbyle de 2 à 30 atomes de carbone, ce reste pouvant être substitué par au moins un groupe alkoxy ou au moins un halogène. A titre d'exemple, et sans que la liste soit limitative, R peut être un reste éthyle, n-propyle, isopropyle, n-butyle, t-butyle, cyclohexyle, benzyle, diphénylméthyle, phényle, méthyl-2-phényle, méthyl-4-phényle, méthoxy-2-phényle, méthoxy-4-phényle, diméthyl-2,6-phényle, diisopropyl-2,6-phényle, t-butyl-2-phényle, t-butyl-2-méthyl-4-phényle, di-t-butyl-2,6-phényle, di-t-butyl-2,6-méthyl-4-phényle, tri-t-butyl-2,4,6-phényle, phényl-2-phényle, diphényl-2,6-phényle, fluoro-2-phényle, fluoro-4-phényle, pentafluorophényle. Dans les amidures R₂N-, R₂ peut constituer avec l'azote un hétérocycle azoté, tel que la pyrrolidine ou la pipéridine. R' est un reste alkyle contenant de 1 à 20 atomes de carbone, de préférence de 1 à 6 atomes de carbone, par exemple méthyle, éthyle, isobutyle, q et r sont égaux à 1 ou 2 de telle façon que la somme q + r soit égale à 3.

On citera comme composés d'aluminium préférés ceux répondant à la formule générale (RO)_{q}AlR'ᵣ, dans laquelle R est un reste hydrocarbyle choisi dans le groupe formé par les restes alkyle, cycloalkyle, alkényle, aryle, aryle ou cycloalkyle substitués, un reste hydrocarbyle de 2 à 30 atomes de carbone, ce reste pouvant être substitué par au moins un groupe alkoxy ou au moins un halogène, les restes aryle et aryle substitué étant préférés. R' est choisi dans le groupe formé par les restes méthyle, éthyle, isobutyle, les restes contenant de 1 à 20 atomes de carbone, les restes contenant de 1 à 6 atomes de carbone.

Les composés d'aluminium plus particulièrement préférés sont choisis dans le groupe formé par le bis-(di-t-butyl-2,6-méthyl-4-phénoxy)-isobutyl-aluminium, le bis-(di-t-butyl-2,6-méthyl-4-phénoxy)-éthyl-aluminium, le bis-(di-t-butyl-2,6-méthyl-4-phénoxy)-méthyl-aluminium.

La préparation des composés X_{q}AlR'ᵣ est connue dans la littérature. Tout procédé de préparation de ces composés convient. Dans le cas des composés (RO)_{q}AlR'ᵣ (cas où X = RO-), on peut par exemple faire réagir un alcool ou un phénol ROH avec un trialkylaluminium AlR'₃ dans un solvant organique, par exemple un hydrocarbure ou un éther.

L'agent stabilisant injecté joue donc le rôle d'agent anti-désactivation, c'est-à-dire qu'il réduit la désactivation du catalyseur, et donc permet d'augmenter la durée de cycle entre deux régénérations du catalyseur, réduisant ainsi considérablement la fréquence de ces régénérations.

L'invention s'applique pour tout catalyseur de métathèse, par exemple les catalyseurs conventionnellement utilisés et comprenant au moins un élément choisi parmi le rhénium, le molybdène et le tungstène, le rhénium étant préféré. Parmi les catalyseurs conventionnels contenant du rhénium, on peut citer les catalyseurs décrits dans les brevets US 4 795 734 et US 5 449 852.

Des catalyseurs de métathèse à base de rhénium beaucoup plus actifs que les catalyseurs conventionnels ont été décrits dans le brevet EP 769 323. Ils comprennent au moins trois composants : un support poreux minéral, 0,01 à 20 % en poids de rhénium sous forme oxyde, et 0,01 à 10 % en poids d'aluminium introduit sous forme d'un composé d'aluminium promoteur de formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que la somme q + r soit égale à 3, un traitement thermique suivant l'imprégnation.

Dans le cas de ces catalyseurs de métathèse non conventionnels à base de rhénium, le composé d'aluminium de formule générale (RO)_{q}AlR'ᵣ est donc utilisé à la fois comme promoteur du catalyseur au cours de sa préparation, et injecté ensuite, de préférence en continu, en tant qu'agent stabilisant de ce catalyseur.

On a repris ci-après la description du catalyseur préféré telle que dans le brevet EP 769 323.

Le support poreux minéral est avantageusement un support à caractère acide ou neutre, plus particulièrement une alumine, une silice ou une silice-alumine, ayant une surface spécifique de 10 à 400 m²/g. De préférence, le support poreux est constitué par de l'alumine ou par un composé contenant au moins 75 % en poids d'alumine, qui doit avantageusement présenter une surface appréciable, par exemple au moins 10 m²/g, et de préférence au moins 50 m²/g, et un volume de pores suffisant, par exemple au moins 0,1 ml/g, et de préférence 0,3-1 ml/g. On peut utiliser par exemple une alumine du même type que celles des catalyseurs de reformage catalytique.

Le précurseur du composé de rhénium utilisé est choisi de préférence dans le groupe formé par l'heptoxyde de rhénium, le perrhénate d'ammonium et l'acide perrhénique. Le composé du rhénium peut être introduit sur le support par exemple par sublimation en phase vapeur ou par imprégnation en solution. On préfère généralement utiliser la méthode d'imprégnation à sec, où le composé du rhénium est mis en solution dans l'eau ou dans un solvant organique, par exemple un hydrocarbure, un alcool ou un éther. On règle la quantité de rhénium sur le support par le choix de la concentration de la solution d'imprégnation, sa quantité étant telle que le volume de cette solution soit égal ou légèrement inférieur au volume poreux du solide à imprégner. Lorsque la quantité de rhénium que l'on désire imprégner est supérieure à celle que permet d'introduire une solution à sa limite de saturation, on doit effectuer l'opération en plusieurs fois, avec des séchages intermédiaires pour éliminer le solvant d'imprégnation, à une température de par exemple 90 à 250 °C, de préférence 100 à 180 °C. Ceci permet d'introduire de 0,01 à 20 %, de préférence de 0,1 à 15 %, et encore plus avantageusement de 0,5 à 8 % en poids de rhénium (exprimé en rhénium métal).

Après l'introduction du précurseur de rhénium sur le support, on effectue un séchage à une température de par exemple 90 à 250 °C, de préférence 100 à 180 °C, puis une calcination à une température de par exemple 250 à 1000 °C, et de préférence 300 à 600 °C, pendant une durée de 10 minutes à 10 heures, et de préférence de 30 minutes à 5 heures. Après calcination, le solide est refroidi sous atmosphère sèche et inerte, par exemple sous azote ou sous argon.

Le composé de l'aluminium promoteur (RO)_{q}AlR'ᵣ peut être introduit sur le support par toutes les méthodes connues de l'homme de l'art, mais il est impératif d'opérer à l'abri de l'air et de l'humidité. On peut imprégner le support par un excès d'une solution contenant le composé de l'aluminium. Après un temps de contact qui peut aller de quelques minutes à quelques jours, on essore le solide et on le lave au solvant pour éliminer la portion du composé qui ne s'est pas fixée. On peut aussi, dans un mode opératoire qui est préféré, utiliser la méthode d'imprégnation à sec. On ajuste alors la concentration en aluminium de la solution en fonction de la quantité d'aluminium que l'on souhaite déposer sur le solide, de façon que le volume de cette solution soit égal ou légèrement inférieur au volume poreux du solide à imprégner. Le solvant mis en oeuvre dans cette imprégnation est de préférence un solvant organique, par exemple un hydrocarbure ou un éther. Ceci permet d'introduire de 0,01 à 10 %, de préférence de 0,05 à 5 %, et encore plus avantageusement de 0,1 à 5 % en poids d'aluminium (exprimé en aluminium métal).

Après introduction du composé de l'aluminium promoteur, la préparation du catalyseur peut être terminée par un séchage, sous vide ou sous un courant de gaz de préférence inerte, à une température de 0 à 1000 °C, de préférence à une température voisine de l'ambiante, de 0 à 50 °C. Aucune opération d'activation, chimique ou thermique, n'est nécessaire pour déclencher l'activité de ces catalyseurs, et la calcination est déconseillée. Il suffit de les mettre en contact avec une oléfine pour que la réaction de métathèse démarre.

Au lieu de préparer le composé (RO)_{q}AlR'ᵣ et de le mettre au contact du catalyseur supporté au rhénium, ainsi que précédemment décrit, on peut mettre ledit catalyseur supporté au rhénium directement en contact des précurseurs du composé (RO)_{q}AlR'ᵣ, qui sont par exemple ROH et AlR'₃. De la même façon que précédemment, la préparation peut se terminer par un séchage.

Dans le procédé de métathèse, les oléfines susceptibles de réagir sont des monooléfines ayant de 2 à 30 atomes de carbone, par exemple l'éthylène, le propylène, les butènes, les pentènes, les hexènes, les octènes, des cyclooléfines ayant de 3 à 20 atomes de carbone, par exemple le cyclopentène, le cyclooctène, le norbornène, des polyoléfines ayant de 4 à 30 atomes de carbone, par exemple l'hexadiène-1,4, l'octadiène-1,7, des cyclopolyoléfines ayant de 5 à 30 atomes de carbone, par exemple le cyclooctadiène-1,5, le norbornadiène, le dicyclopentadiène.

D'autres oléfines susceptibles de réagir en métathèse sont les monooléfines ou les polyoléfines, linéaires ou cycliques, portant des groupes fonctionnels comme par exemple des halogènes, des éthers, des nitriles, des amines, des amides, des silanes ou des groupes ester tels que l'oléate de méthyle. Le procédé peut également mettre en oeuvre en cométathèse un mélange des oléfines précédentes.

Le procédé de métathèse selon l'invention s'applique plus particulièrement au rééquilibrage entre elles des oléfines légères issues du craquage à la vapeur ou du craquage catalytique (FCC), telles que l'éthylène, le propylène, les butènes ou les pentènes. Il permet par exemple de produire du propylène à partir d'éthylène et d'une coupe C₄ oléfinique riche en butène-2, telle qu'un raffinat-2 de vapocraquage préalablement soumis à une isomérisation après enlèvement ou transformation du butadiène et de l'isobutène. On peut aussi produire du propylène en deux étapes à partir d'une charge contenant du butène-1 et du butène-2 qui donne dans une première étape du propylène et du pentène-2, ce dernier étant dans une deuxième étape mis en contact avec de l'éthylène pour donner de nouveau du propylène ainsi que du butène-1. Il permet aussi de produire un mélange de propylène, d'isobutène, et de n-butènes riches en butène-1, à partir d'éthylène et d'une coupe C₅ enrichie en pentène-2 et méthyl-2 butène-2. Il permet également de produire un mélange d'isobutène et de n-butènes riches en butène-1 à partir de propylène et d'une coupe C₅ enrichie en pentène-2 et méthyl-2 butène-2.

Le procédé selon l'invention peut être mis en oeuvre avec un catalyseur disposé en lit fixe, en lit mobile ou en lit agité, en lit fluidisé. Le catalyseur est avantageusement préparé ex-situ et donc introduit préparé dans le réacteur. Le procédé peut être réalisé en batch, en continu, ou en mode discontinu, par exemple lorsque l'écoulement du catalyseur en lit mobile est interrompu pour une étape du procédé.

Le procédé peut aussi être mis en oeuvre par distillation réactive, la réaction catalytique et la distillation des réactifs et des produits ayant lieu simultanément dans la colonne.

La colonne de distillation réactive employée dans le procédé selon l'invention peut être de n'importe quel type. Dans une disposition préférée, au moins une zone contenant le catalyseur est aménagée. La disposition mécanique du catalyseur dans la ou les zones catalytiques doit être telle qu'elle gêne le moins possible les flux de vapeur et de liquide entre les deux zones de séparation qui l'encadrent. En même temps le catalyseur doit être disposé de sorte qu'une surface suffisante soit exposée pour catalyser convenablement la réaction de métathèse. Le catalyseur peut par exemple être disposé en vrac ou en couche mince sur des plateaux perforés ou sur des grilles, ou dans des sachets suspendus ou posés sur des supports assurant leur tenue mécanique, ou de toute autre façon connue de l'homme de l'art. D'autre part, le catalyseur peut être disposé dans la colonne de manière à n'être traversé que par un flux ascendant de phase liquide. Il peut également être disposé sous forme de garnissage catalytique, suivant les différents modes de mise en oeuvre connus (tel que garnissage structuré). Les zones de séparation qui encadrent les zones catalytiques peuvent comporter des plateaux ou du garnissage. Toutes les technologies dérivées de celles-ci sont ici incluses.

A titre d'exemple de colonnes, on citera celles décrites dans les documents EP-A-0 332 525, FR-A-2 628 737, FR-A-2 684 893, US-5 221 441 et EP-466 954.

La colonne de distillation réactive est opérée dans des conditions telles qu'il s'y produit à la fois la réaction de métathèse et un fractionnement entre les diverses oléfines. Par exemple, la pression dans la colonne sera ajustée en fonction des points d'ébullition des réactifs et des produits, et de la température optimale d'utilisation du catalyseur. La température opératoire peut varier de -20 à 200 °C, de préférence de 10 à 100 °C, et la pression peut varier de 0,01 à 10 MPa, de préférence de 0,1 à 5 MPa.

Ces conditions sont également utilisables sur les autres types de réacteur pour effectuer la métathèse.

Selon l'invention, l'agent stabilisant est injecté directement (séparément) dans le réacteur, ou dans la charge entrant au réacteur, ou plus généralement dans un réactif ou un flux de recyclage entrant dans le réacteur, ou encore avec le catalyseur en circulation. L'injection est réalisée de façon continue ou discontinue selon les besoins. Elle a lieu pendant tout le temps de la réaction de métathèse. En règle générale, on introduira dans le milieu réactionnel de 0,01 à 20 %, et de préférence de 0,01 à 5 % en poids, d'agent stabilisant, et en particulier du composé d'aluminium (RO)_{q}AlR'ᵣ, comptés par rapport au flux entrant au réacteur.

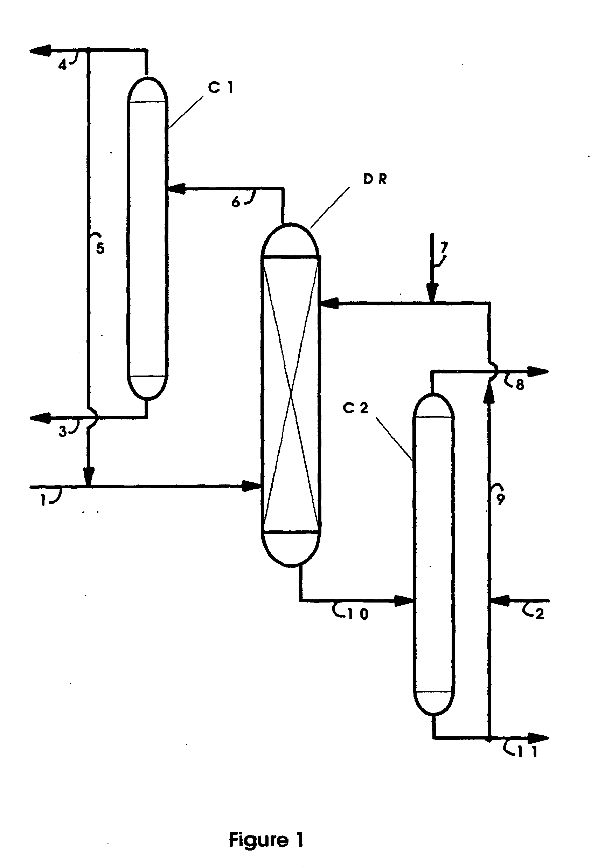

A titre illustratif, le procédé est maintenant décrit à partir de la figure 1, pour la production de propylène à partir d'éthylène et d'une coupe C₄ oléfinique riche en butène-2 dans une mise en oeuvre avec distillation réactive.

La colonne réactive (DR) est par exemple agencée avec des zones de réactions et de séparations alternées. Elle est alimentée par les réactifs de telle façon que ceux-ci se croisent naturellement à l'intérieur en raison de leurs points d'ébullition respectifs. Ainsi l'éthylène frais (réactif) est injecté par le conduit (1) dans le bas de la colonne réactive (DR) tandis que la charge contenant la coupe C₄ oléfinique riche en butène-2 est introduite dans le flux de recyclage (conduit (9) arrivant dans la zone haute de la colonne) par le conduit (2). L'agent stabilisant est introduit par un conduit (7) dans le conduit (9) et est ainsi injecté avec la charge.

En tête de la colonne réactive (DR) sort un mélange éthylène-propylène par le conduit (6). Ce mélange est séparé dans la colonne de distillation (C1), d'où sort en tête par le conduit (5) un flux d'éthylène qui est renvoyé en bas de la colonne (DR) après mélange avec l'éthylène frais du conduit (1). Une petite purge pour assurer l'élimination des traces d'éthane toujours présentes dans l'éthylène est effectuée par le conduit (4). Le propylène produit est évacué par le conduit (3) en bas de la colonne (C1).

En fond de la colonne réactive (DR) sort par le conduit (10) une coupe C₄⁺ comprenant l'ensemble des C₄ saturés et oléfiniques qui n'ont pas réagi, les oléfines sous-produits en C₅ et C₆, ainsi que l'agent stabilisant.

Une petite colonne auxiliaire (C2) alimentée par le conduit (10) sépare en tête par le conduit (8) une purge C₄ évitant l'accumulation d'isobutane et d'isobutène, et en fond par le conduit (9) un flux de recyclage C₄. Ce flux contenant des butènes est recyclé vers la colonne (DR). Une purge de l'agent stabilisant, dont en général au moins une partie est dégradée et doit être remplacée, du n-butane et des oléfines sous-produits en C₅ et C₆ est assurée par le conduit (11 ).

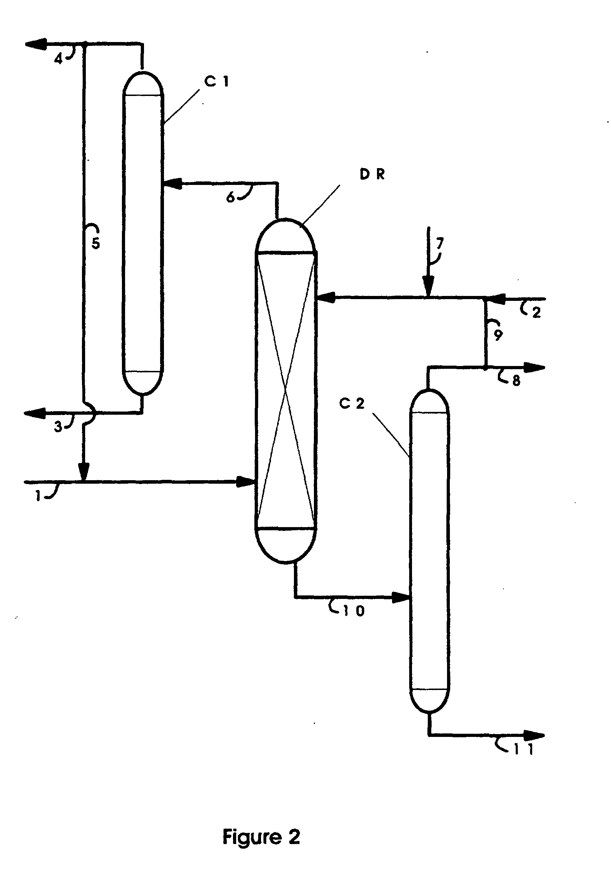

Dans une variante de ce schéma, illustrée par la figure 2, on peut ne pas recycler l'agent stabilisant. Le flux de recyclage des C₄ assuré par le conduit (9) est alors pris sur le conduit (8) en tête de la colonne (C2), dont le fonctionnement doit être adapté à cette situation. Ce flux de recyclage peut aussi être assuré par un soutirage latéral dans la colonne (C2). Le conduit (11) en fond de la colonne (C2) n'assure plus qu'une fonction de purge de l'agent stabilisant, du n-butane et des oléfines sous-produits en C₅ et C₆. La fonction du conduit (8) est inchangée, de même que le reste du schéma.

Les exemples suivants illustrent l'invention sans en limiter la portée.

### EXEMPLE 1 (comparatif)

### Préparation du catalyseur :

Dans une première étape, on calcine à 300 °C sous air une alumine gamma cubique ayant une surface spécifique de 184 m²/g et un volume poreux de 0,67 ml/g. Après refroidissement à température ambiante, on prélève 10 g d'alumine calcinée. On prépare une solution pour l'imprégnation du rhénium en diluant 0,24 ml d'une solution aqueuse concentrée d'acide perrhénique contenant 54 % en poids de rhénium (masse spécifique : 2,4 g/ml) dans 5 ml d'eau. Cette solution est imprégnée sur les 10 g d'alumine prélevés. Après 30 minutes de contact à température ambiante, on sèche le solide obtenu dans une étuve à 120 °C pendant une nuit. On le calcine ensuite sous un courant d'air (environ 20 l/h) séché par passage à travers un lit de tamis moléculaire, à une température de 550 °C pendant 2 heures. Pendant la période ultérieure de refroidissement, on substitue au courant d'air un courant d'azote sec. Le solide obtenu est conservé et manipulé en atmosphère d'azote sec. Sa teneur en rhénium métal est de 3 % en poids.

Dans un ballon de 250 ml placé sous atmosphère d'argon et muni d'un barreau magnétique, on introduit une solution de 0,493 g de triisobutylaluminium dans 20 ml de pentane, puis on injecte goutte-à-goutte, sous agitation et à température ambiante, une solution de 1,095 g de di-t-butyl-2,6-méthyl-4-phénol dans 30 ml de pentane. Après environ 30 heures de réaction, le pentane est évaporé sous vide et l'analyse du solide blanc restant indique qu'il est constitué essentiellement par le bis-(di-t-butyl-2,6-méthyl-4-phénoxy)-isobutylaluminium. Ce composé est remis en solution dans 5 ml d'heptane.

La solution dans l'heptane du bis-(di-t-butyl-2,6-méthyl-4-phénoxy)-isobutylaluminium est alors imprégnée sur le solide contenant le rhénium obtenu dans la première étape. Après environ 30 minutes de contact, l'heptane absorbé sur le solide est éliminé par évaporation sous vide à température ambiante. On obtient ainsi un catalyseur de métathèse contenant 3 % en poids de rhénium et 0,67 % en poids d'aluminium (en sus de l'aluminium inclus dans l'alumine), qui est conservé en atmosphère sèche et inerte avant utilisation.

### Utilisation en métathèse du propylène :

Les exemples 1 et 2 se rapportent à la métathèse du propylène pour donner de l'éthylène et du butène-2. Cette réaction facile à mettre en oeuvre puisqu'elle ne nécessite qu'un seul réactif, est l'inverse de la réaction décrite dans l'exemple 3 qui produit du propylène à partir d'éthylène et de butène-2. La désactivation du catalyseur est la même dans ces deux réactions.

Dans un réacteur constitué par un tube en acier inoxydable muni d'une double enveloppe avec circulation d'eau permettant la régulation de la température, on charge en lit fixe, à l'abri de l'air et de l'humidité, le catalyseur préparé ci-dessus. Du propylène liquide est injecté au moyen d'une pompe par le bas du réacteur, avec un débit de 49,6 g/h. La température est réglée à 35 °C et la pression est maintenue à 3,5 MPa au moyen d'un régulateur placé en aval du réacteur. Dans ces conditions, la conversion du propylène à la sortie du réacteur est initialement de 30 %, en un mélange équimolaire d'éthylène et de butène-2. Elle évolue au cours du temps comme indiqué dans le tableau 1. Le catalyseur a perdu la moitié de son activité au bout de 30 heures.

### EXEMPLE 2

### Préparation du catalyseur :

Un second lot de 10 g de catalyseur est préparé suivant le mode opératoire décrit dans l'exemple 1.

### Utilisation en métathèse du propylène :

On utilise le même réacteur et les mêmes procédures que dans l'exemple 1. Mais à la différence de l'exemple 1, la charge de métathèse est constituée par du propylène liquide mélangé avec 0,24 % en poids de bis-(di-t-butyl-2,6-méthyl-4-phénoxy)-isobutylaluminium. Le bis-(di-t-butyl-2,6-méthyl-4-phénoxy)-isobutylaluminium a été préparé comme décrit dans l'exemple 1. La charge de métathèse est injectée au moyen d'une pompe par le bas du réacteur, avec un débit de 49,6 g/h. La température est réglée à 35 °C et la pression est maintenue à 3,5 MPa. Dans ces conditions, la conversion du propylène à la sortie du réacteur est initialement de 30 %, en un mélange équimolaire d'éthylène et de butène-2. Elle évolue au cours du temps comme indiqué dans le tableau 1. Le catalyseur a perdu seulement 16 % de son activité au bout de 30 heures.

**Tableau 1**

| Temps (h) | Conversion du propylène (%) | |
|---|---|---|
| | Exemple 1 | Exemple 2 |
| 0 | 30 | 30 |
| 10 | 25,8 | 28,9 |
| 20 | 20,3 | 27,4 |
| 30 | 14,6 | 25,6 |

Cet exemple illustre l'effet bénéfique apporté par l'injection en continu avec la charge du bis-(di-t-butyl-2,6-méthyl-4-phénoxy)-isobutylaluminium.

### EXEMPLE 3

La réaction de métathèse est conduite en colonne de distillation réactive. La colonne est constituée par un fût en acier inoxydable d'un diamètre interne de 5 cm et de 250 cm de hauteur, rempli de trois lits de garnissage, soit, dans l'ordre à partir du bas : un lit de garnissage de distillation sur 100 cm de hauteur, un lit de garnissage catalytique sur 50 cm et un lit de garnissage de distillation sur 100 cm. Chaque lit est soutenu par une grille en forme de V, rendue solidaire du fût.

Le garnissage de distillation est un garnissage non structuré, constitué d'éléments métalliques ayant l'apparence de ressorts sensiblement hélicoïdaux à spires jointives, d'une longueur de 3 mm et d'un diamètre de 1 mm, connu sous le nom de Dixon, et réputé très efficace en distillation.

Le garnissage catalytique est composé du garnissage de distillation ci-dessus défini et de catalyseur préparé suivant le mode opératoire décrit dans l'exemple 1. Le garnissage catalytique est obtenu par mélange de ces deux composants, à raison de un volume de catalyseur sous forme de billes d'un diamètre moyen égal à 2 mm, et de 25 volumes de garnissage de distillation. Ce mélange est brassé manuellement dans un récipient en verre de manière à rendre homogène la répartition du catalyseur dans le garnissage de distillation.

La colonne est reliée, en pied, à un bouilleur chauffé électriquement et, en tète, à un condenseur et un ballon de reflux. Elle est rendue adiabatique par compensation des pertes thermiques, au moyen de dix éléments chauffants disposés sur toute sa hauteur. Chaque élément chauffant est commandé séparément pour que la température au voisinage immédiat de la paroi externe de la colonne soit égale à celle qui s'établit à l'intérieur de la colonne, à la même cote. Par ailleurs, la colonne est munie des capteurs et instruments nécessaires à la régulation des paramètres opératoires.

La colonne ainsi équipée est mise en fonctionnement en régime continu, sous une pression de 2 Mpa. Elle est alimentée au niveau du garnissage catalytique, à raison de 265 g/h, par une coupe oléfinique constituée principalement de butène-2 (77,3 % en poids) et de butane, dans laquelle on a dissout du bis-(di-t-butyl-2,6-méthyl-4-phénoxy)-isobutyl-aluminium à raison de 0,24 % en poids. Le bis-(di-t-butyl-2,6-méthyl-4-phénoxy)-isobutylaluminium a été préparé comme décrit dans l'exemple 1. Au niveau bas du garnissage catalytique, la colonne reçoit de l'éthylène avec un débit de 670 g/h. La chauffe de la colonne est régulée de manière à séparer en tête l'éthylène et le propylène. Le taux de reflux est établi à 1,8.

Dans ces conditions, après mise en régime de la colonne, on recueille d'une part, 870 g/h de distillat constitué principalement du propylène formé (34,4 % en poids) et de l'éthylène qui n'a pas réagi, et d'autre part, 61 g/h de résidu constitué par le butane qui est resté inerte dans la réaction, et par l'agent stabilisant.

Le distillat recueilli, redistillé suivant une procédure connue, donnerait finalement 300 g/h de propylène et 570 g/h d'éthylène qui serait à recycler dans la colonne de distillation réactive.

## Revendications

1. Procédé de métathèse d'oléfines en présence d'un catalyseur et d'un agent stabilisant injecté dans le milieu réactionnel pour réduire la désactivation du catalyseur, l'injection est réalisée de façon continue ou discontinue pendant tout le temps de la réaction de métathèse, ledit procédé étant **caractérisé en ce que** :
- les oléfines susceptibles de réagir sont des monooléfines ayant de 2 à 30 atomes de carbone, des cyclooléfines ayant de 3 à 20 atomes de carbone, des polyoléfines ayant de 4 à 30 atomes de carbone et des cyclopolyoléfines ayant de 5 à 30 atomes de carbone ;
- l'agent stabilisant injecté dans le milieu réactionnel est un composé de l'aluminium X_{q}AlR'ᵣ, dans lequel X est un radical choisi dans le groupe formé par les alkoxydes et les aryloxydes RO-, les sulfures RS- et les amidures R₂N-, R est un reste hydrocarbyle contenant de 1 à 40 atomes de carbone, R' est un reste alkyle contenant de 1 à 20 atomes de carbone, q et r sont égaux à 1 ou 2 de telle façon que la somme q+r soit égale à 3 ; et
- le catalyseur contient du rhénium.

2. Procédé de métathèse des oléfines selon la revendication 1, **caractérisé en ce que** l'agent stabilisant du catalyseur répond à la formule générale (RO)_{q}AlR'ᵣ, dans laquelle R est un reste hydrocarbyle choisi dans le groupe formé par les restes alkyle, cycloalkyle, alkényle, aryle, aryle ou cycloalkyle substitués, un reste hydrocarbyle de 1 à 40 atomes de carbone, ce reste pouvant être substitué par au moins un groupe alkoxy ou au moins un halogène, et R' est choisi dans le groupe formé par les restes méthyle, éthyle, isobutyle, les restes contenant de 1 à 20 atomes de carbone, les restes contenant de 1 à 6 atomes de carbone.

3. Procédé de métathèse des oléfines selon l'une des revendications précédentes, **caractérisé en ce que** l'agent stabilisant répond à la formule générale (RO)_{q}AlR'ᵣ, dans laquelle R est un reste hydrocarbyle choisi dans le groupe formé par les restes aryle ou aryle substitué.

4. Procédé de métathèse des oléfines selon l'une des revendications précédentes, **caractérisé en ce que** l'agent stabilisant est choisi dans le groupe formé par le bis-(di-t-butyl-2,6-méthyl-4-phénoxy)-isobutyl-aluminium, le bis-(di-t-butyl-2,6-méthyl-4-phénoxy)-éthyl-aluminium, le bis-(di-t-butyl-2,6-méthyl-4-phénoxy)-méthyl-aluminium.

5. Procédé selon l'une des revendications précédentes, **caractérisé en ce que** le procédé est réalisé avec un catalyseur en lit fixe.

6. Procédé selon l'une des revendications 1 à 4, **caractérisé en ce que** le procédé est réalisé avec un catalyseur en lit mobile ou en lit agité.

7. Procédé selon l'une des revendications 1 à 4, **caractérisé en ce que** le procédé est réalisé avec un catalyseur en lit fluidisé.

8. Procédé selon l'une des revendications 1 à 4, **caractérisé en ce que** le procédé est réalisé dans une colonne de distillation réactive.

9. Procédé selon l'une des revendications 1 à 4, **caractérisé en ce que** le procédé est réalisé dans une colonne de distillation réactive, dans laquelle le catalyseur est disposé de manière à n'être traversé que par un flux ascendant de phase liquide.

10. Procédé de métathèse des oléfines selon l'une des revendications précédentes, **caractérisé en ce que** l'agent stabilisant du catalyseur est mélangé avec la charge oléfinique.

11. Procédé de métathèse des oléfines selon l'une des revendications 8 à 10, **caractérisé en ce que** l'agent stabilisant du catalyseur est injecté séparément dans la colonne de distillation réactive.

12. Procédé de métathèse des oléfines selon l'une des revendications précédentes, **caractérisé en ce que** le catalyseur contient au moins un élément choisi dans le groupe formé par le molybdène, le tungstène, le rhénium.

13. Procédé de métathèse des oléfines selon l'une des revendications précédentes, **caractérisé en ce que** le catalyseur comprend un support poreux minéral, 0,01 à 20 % en poids de rhénium sous forme oxyde, et 0,01 à 10 % en poids d'aluminium introduit sous forme d'un composé d'aluminium promoteur de formule générale (RO)_{q}AlR'ᵣ, dans laquelle R est un reste hydrocarbyle contenant de 1 à 40 atomes de carbone, R' est un reste alkyle contenant de 1 à 20 atomes de carbone, q et r sont égaux à 1 ou 2 de telle façon que la somme q + r soit égale à 3.

14. Procédé de métathèse des oléfines selon l'une des revendications précédentes, **caractérisé en ce que** une charge composée d'éthylène et d'une coupe C₄ riche en butène-2 est mise en contact avec le catalyseur pour produire du propylène.

15. Procédé de métathèse des oléfines selon l'une des revendications précédentes, **caractérisé en ce que** une charge composée d'éthylène et d'une coupe C₅ enrichie en pentène-2 et en méthyl-2-butène-2 est mise en contact avec le catalyseur pour produire du propylène, de l'isobutène et des n-butènes.

16. Procédé de métathèse des oléfines selon l'une des revendications précédentes, **caractérisé en ce que** une charge composée de propylène et d'une coupe C₅ enrichie en pentène-2 et en méthyl-2-butène-2 est mise en contact avec le catalyseur pour produire de l'isobutène et des n-butènes.

17. Procédé de métathèse des oléfines selon l'une des revendications précédentes, **caractérisé en ce qu'**il fonctionne à une température comprise entre -20 et 200 °C et sous une pression de 0,01 à 10 MPa.

18. Procédé de métathèse des oléfines selon l'une des revendications précédentes, **caractérisé en ce qu'**il est ajouté pendant la réaction 0,01 à 20 % pds d'agent stabilisant (compté par rapport au flux entrant dans le réacteur).

## Patentansprüche

1. Verfahren zur Olefin-Metathese in Anwesenheit eines Katalysators und eines Stabilisierungsmittels, das während der gesamten Reaktionsdauer der Metathese kontinuierlich oder diskontinuierlich in das Reaktionsmedium injiziert wird, um die Deaktivierung des Katalysators zu vermindern; wobei das Verfahren **dadurch gekennzeichnet ist, dass**:
- die reaktionsfähigen Olefine Monoolefine mit 2 bis 30 Kohlenstoffatomen, Cycloolefine mit 3 bis 20 Kohlenstoffatomen, Polyolefine mit 4 bis 30 Kohlenstoffatomen und Cyclopolyolefine mit 5 bis 30 Kohlenstoffatomen sind;
- das Stabilisierungsmittel, das in das Reaktionsmedium injiziert wird, eine Aluminiumverbindung X_{q}AlR'ᵣ ist, in der X ein Radikal ist, ausgewählt aus der Gruppe, bestehend aus RO-Alkoxiden und -Aryloxiden, RS-Sulfiden und R₂N-Amiden, R ein Hydrocarbylrest mit 1 bis 40 Kohlenstoffatomen ist, R' ein Alkylrest mit 1 bis 20 Kohlenstoffatomen ist, q und r gleich 1 bzw. 2 sind, so dass die Summe von q+r 3 ergibt; und
- der Katalysator Rhenium enthält.

2. Verfahren zur Olefin-Metathese nach Anspruch 1, **dadurch gekennzeichnet, dass** das Stabilisierungsmittel des Katalysators der allgemeinen Formel (RO)_{y}AlR'ᵣ entspricht, in der R ein Hydrocarbylrest ist, ausgewählt aus der Gruppe bestehend aus Alkyl-, Cycloalkyl-, Alkenyl-, Arylresten, substituiertem Aryl oder Cycloalkyl, einem Hydrocarbylrest mit 1 bis 40 Atomen, wobei dieser Rest durch mindestens eine Alkoxygruppe oder mindestens ein Halogen substituiert werden kann, und R' ausgewählt ist aus der Gruppe bestehend aus Methyl-, Ethyl-, Isobutylresten, Resten mit 1 bis 20 Kohlenstoffatomen, Resten mit 1 bis 6 Kohlenstoffatomen.

3. Verfahren zur Olefin-Metathese nach einem der vorhergehenden Ansprüche, **dadurch gekennzeichnet, dass** das Stabilisierungsmittel der allgemeinen Formel (RO)_{q}AlR'ᵣ entspricht, in der R ein Hydrocarbylrest ist, ausgewählt aus der Gruppe, bestehend aus Arylresten oder substituiertem Aryl.

4. Verfahren zur Olefin-Metathese nach einem der vorhergehenden Ansprüche, **dadurch gekennzeichnet, dass** das Stabilisierungsmittel ausgewählt ist aus der Gruppe, bestehend aus Bis-(di-t-butyl-2,6-methyl-4-phenoxy)-isobutylaluminium, Bis-(di-t-butyl-2,6-methyl-4-phenoxy)-ethylaluminium, Bis-(di-t-butyl-2,6-methyl-4-phenoxy)-methylaluminium.

5. Verfahren nach einem der vorhergehenden Ansprüche, **dadurch gekennzeichnet, dass** das Verfahren mit einem Katalysator im Festbett ausgeführt wird.

6. Verfahren nach einem der Ansprüche 1 bis 4, **dadurch gekennzeichnet, dass** das Verfahren mit einem Katalysator im Wirbelbett oder im Fließbett ausgeführt wird.

7. Verfahren nach einem der Ansprüche 1 bis 4, **dadurch gekennzeichnet, dass** das Verfahren mit einem Katalysator im Fließbett ausgeführt wird.

8. Verfahren nach einem der Ansprüche 1 bis 4, **dadurch gekennzeichnet, dass** das Verfahren in einer reaktiven Destillationskolonne ausgeführt wird.

9. Verfahren nach einem der Ansprüche 1 bis 4, **dadurch gekennzeichnet, dass** das Verfahren in einer reaktiven Destillationskolonne ausgeführt wird, in welcher der Katalysator so angeordnet ist, dass er nur von einem aufsteigenden Flux der Flüssigphase durchquert wird.

10. Verfahren zur Olefin-Metathese nach einem der vorhergehenden Ansprüche, **dadurch gekennzeichnet, dass** das Stabilisierungsmittel des Katalysators mit der Olefinmasse vermischt wird.

11. Verfahren zur Olefin-Metathese nach einem der Ansprüche 8 bis 10, **dadurch gekennzeichnet, dass** das Stabilisierungsmittel des Katalysators getrennt in die reaktive Destillationskolonne injiziert wird.

12. Verfahren zur Olefin-Metathese nach einem der vorhergehenden Ansprüche, **dadurch gekennzeichnet, dass** der Katalysator mindestens ein Element enthält, ausgewählt aus der Gruppe bestehend aus Molybdän, Wolfram und Rhenium.

13. Verfahren zur Olefin-Metathese nach einem der vorhergehenden Ansprüche, **dadurch gekennzeichnet, dass** der Katalysator ein poröses mineralisches Substrat, 0,01 bis 20 Gewichtprozent Rhenium in Oxidform und 0,01 bis 10 Gewichtprozent Aluminium enthält, wobei Letzteres zugeführt ist in der Form einer Aluminiumpromoterverbindung der allgemeinen Formel (RO)_{q}AlR'ᵣ, in der R ein Hydrocarbylrest mit 1 bis 40 Kohlenstoffatomen ist, R' ein Alkylrest mit 1 bis 20 Kohlenstoffatomen ist, q und r gleich 1 bzw. 2 sind, so dass die Summe von q+r 3 ergibt.

14. Verfahren zur Olefin-Metathese nach einem der vorhergehenden Ansprüche, **dadurch gekennzeichnet, dass** eine Masse, bestehend aus Ethylen und einer Schale C₄, das reich an Buten-2 ist, mit dem Katalysator in Kontakt gebracht wird, um Propylen zu produzieren.

15. Verfahren zur Olefin-Metathese nach einem der vorhergehenden Ansprüche, **dadurch gekennzeichnet, dass** eine Masse, bestehend aus Ethylen und einer Schale C₄, angereichert mit Penten-2 und Methyl-2-buten-2 mit dem Katalysator in Kontakt gebracht wird, um Propylen, Isobuten und n-Butene zu produzieren.

16. Verfahren zur Olefin-Metathese nach einem der vorhergehenden Ansprüche, **dadurch gekennzeichnet, dass** eine Masse, bestehend aus Propylen und einer Schale C₅, angereichert mit Penten-2 und Methyl-2-buten-2 mit dem Katalysator in Kontakt gebracht wird, um Isobuten und n-Butene zu produzieren.

17. Verfahren zur Olefin-Metathese nach einem der vorhergehenden Ansprüche, **dadurch gekennzeichnet, dass** es bei einer Temperatur zwischen -20 und 200 °C und unter einem Druck von 0,01 bis 10 MPa funktioniert.

18. Verfahren zur Olefin-Metathese nach einem der vorhergehenden Ansprüche, **dadurch gekennzeichnet, dass** während der Reaktion 0,01 bis 20 Gewichtprozent des Stabilisierungsmittels (gezählt in Bezug auf den in den Reaktor einlaufenden Flux) hinzugefügt werden.

## Claims

1. Process for metathesis of olefins in the presence of a catalyst and a stabilizing agent that is injected into the reaction medium to reduce the deactivation of the catalyst, said stabilizing agent is injected during the entire course of the metathesis process, whereby the injection takes place continuously or discontinuously, said process being **characterized in that** :
- the olefins that are able to react are monoolefins that have 2 to 30 carbon atoms, cycloolefins that have 3 to 20 carbon atoms, polyolefins that have 4 to 30 carbon atoms, and cyclopolyolefins that have 5 to 30 carbon atoms
- said stabilizing agent which is injected into the reaction medium is an aluminum compound X_{q}AlR'ᵣ -- in which X is a radical that is selected from the group that is formed by alkoxides and aryloxides RO-, sulfides RS- and amides R₂N-; R is a hydrocarbyl radical that contains 1 to 40 carbon atoms; R' is an alkyl radical that contains 1 to 20 carbon atoms; q and r are equal to 1 or 2 so that the sum of q + r is equal to 3
- and the catalyst contains rhenium.

2. Process for metathesis of olefins according to claim 1, wherein the stabilizing agent of the catalyst corresponds to general formula (RO)_{q}AlR'ᵣ, in which R is a hydrocarbyl radical that is selected from the group that is formed by the alkyl, cycloalkyl, alkenyl, aryl, substituted aryl or cycloalkyl radicals, a hydrocarbyl radical of 1 to 40 carbon atoms, whereby this radical can be substituted by at least one alkoxy group or at least one halogen, and R' is selected from the group that is formed by the methyl, ethyl, isobutyl radicals, whereby the radicals contain 1 to 20 carbon atoms and whereby the radicals contain 1 to 6 carbon atoms.

3. Process for metathesis of the olefins according to one of the preceding claims, wherein the stabilizing agent corresponds to general formula (RO)_{q}AlR'ᵣ, in which R is a hydrocarbyl radical that is selected from the group that is formed by the aryl or substituted aryl radicals.

4. Process for metathesis of the olefins according to one of the preceding claims, wherein the stabilizing agent is selected from the group that is formed by bis-(di-t-butyl-2,6-methyl-4-phenoxy)-isobutylaluminum, bis-(di-t-butyl-2,6-methyl-4-phenoxy)-ethyl-aluminum, bis-(di-t-butyl-2,6-methyl-4-phenoxy)-methyl-aluminum.

5. Process according to one of the preceding claims, wherein the process is carried out with a fixed-bed catalyst.

6. Process according to one of claims 1 to 4, wherein the process is carried out with a fluid-bed or stirred-bed catalyst.

7. Process according to one of claims 1 to 4, wherein the process is carried out with a fluidized-bed catalyst.

8. Process according to one of claims 1 to 4, wherein the process is carried out in a reactive distillation column.

9. Process according to one of claims 1 to 4, wherein the process is carried out in a reactive distillation column, in which the catalyst is placed so as to be traversed only by a rising flow of liquid phase.

10. Process for metathesis of olefins according to one of the preceding claims, wherein the stabilizing anent of the catalyst is mixed with the olefinic feedstock.

11. Process for metathesis of the olefins according to one of claims 8 to 10, wherein the stabilizing agent of the catalyst is injected separately into the reactive distillation column.

12. Process for metathesis of the olefins according to one of the preceding claims, wherein the catalyst contains at least one element that is selected from the group that is formed by molybdenum, tungsten, rhenium.

13. Process for metathesis of olefins according. to one of the preceding claims, wherein the catalyst comprises a porous mineral substrate, 0.01 to 20% by weight of rhenium in oxide form, and 0.01 to 10% by weight of aluminum that is introduced in the form of a promoter aluminum compound of general formula (RO)_{q}AlR'ᵣ, in which R is a hydrocarbyl radical that contains 1 to 40 carbon atoms, R' is an alkyl radical that contains 1 to 20 carbon atoms, q and r are equal to 1 or 2 such that the sum of q + r is equal to 3.

14. Process for metathesis of olefins according to one of the preceding claims, wherein a feedstock that consists of ethylene and a butene-2-rich C₄ fraction is brought into contact with the catalyst to produce propylene.

15. Process for metathesis of olefins according to one of the preceding claims, wherein a feedstock that consists of ethylene and a C₅ fraction that is enriched with pentene-2 and methyl-2-butene-2 is brought into contact with the catalyst to produce propylene, isobutene and n-butenes.

16. Process for metathesis of olefins according to one of the preceding claims, wherein a feedstock that consists of propylene and a C₅ fraction that is enriched with pentene-2 and methyl-2-butene-2 is brought into contact with the catalyst to produce isobutene and n-butenes.

17. Process for metathesis of olefins according to one of the preceding claims, wherein it operates at a temperature of between -20 and 200°C and under a pressure of 0.01 to 10 MPa.

18. Process for metathesis of olefins according to one of the preceding claims, wherein 0.01 to 20% by weight of stabilizing agent (counted relative to the flow that enters the reactor) is added during the reaction.
